Europäisches Patentamt

(19) European Patent Office

Office européen des brevets

(11) Publication number: **0 039 518**
**A1**

## (12) EUROPEAN PATENT APPLICATION

(21) Application number: **81103420.6**

(22) Date of filing: **06.05.81**

(51) Int. Cl.³: **C 12 P 7/06**

(30) Priority: **07.05.80 PH 23985**
**25.09.80 PH 24632**

(43) Date of publication of application: **11.11.81**
**Bulletin 81/45**

(84) Designated Contracting States: **AT BE CH DE FR GB IT LI LU NL SE**

(71) Applicant: **Alvero, Jesus S., No. 1 Matimyas Street cor. Rodriguez, Quezon City (PH)**

(72) Inventor: **Alvero, Jesus S., No. 1 Matimyas Street cor. Rodriguez, Quezon City (PH)**

(74) Representative: **Patentanwälte Beetz sen. - Beetz jr. - Heldrich - Timpe - Siegfried - Schmitt-Fumian, Steinsdorfstrasse 10, D-8000 München 22 (DE)**

(54) **System and process of producing near absolute alcohol.**

(57) As a simple and compact system of producing alcohol continuously and in a shorter time there is provided a system, comprising a sterilizer (1) with pressure, temperature and level monitoring devices (7, 9, 12), a fermentation and destillation tank (2) with a vacuum pump (21) and similar monitoring devices (24 - 27), said tank (2) receiving and storing the sterilized mash for fermentation and destillation under vacuum to produce alcohol vapor, a condenser (3) in communication with said tank (2) and receiving said alcohol vapor and converting it into liquid alcohol, and a collecting tank (4) in communication with said condenser (3) for storing the liquid alcohol, said collecting tank (4) having pressure and level monitoring devices (32, 34), a relief valve (33) for discharging carbon dioxide and an alcohol outlet (35).

The raw materials are heated under controlled conditions; simultaneously sterilized mash is fermented and destilled under partial vacuum to accumulate alcohol vapor and convert it to liquid alcohol.

0039518

# System and process of producing near absolute alcohol

## SPECIFICATION

This invention relates to a system and a process of producing near absolute alcohol.

The traditional process of alcohol production from fermentable sugars and starches involved four separate steps, namely: sterilization, fermentation, distillation and concentration. These steps had to be separated as characteristic conditions of one step is detrimental to the functions of the others, so that the time consuming batch system is mandatory.

For instance, heat that was used in distillation step was fatal to the fermentation agent, and alcohol content in fermentation step cannot go over 15% as the fermentation agent will also die. These two steps of incompatability forced the industry to adopt the cumbersome and time consuming batch system.

The present on-going and further aggravating energy crisis makes alcohol an attractive alternate to replace the fossil fuels, and consequently, the need for a better system and process of producing alcohol is of high priority.

The object therefore of this invention is to provide a simple and compact system of producing alcohol, reducing its physical requirements and cost of production.

Another object of this invention is to provide a system which shortens the process of producing alcohol.

Another object of this invention is to provide a system of producing alcohol which is easy to operate and highly selective in its distillation action.

A further object of this invention is to provide a continuous process of producing alcohol allowing its steps to overlap and made to take place simultaneously without interruption.

Other objects and advantages of this invention will become clear and apparent as this specification proceeds.

In the drawings:

Fig. 1 is a side elevational view of the system for producing alcohol;

Fig. 2 is a top view of the same system; and

Fig. 3 is also a side elevational view of the system with some of its parts broken away.

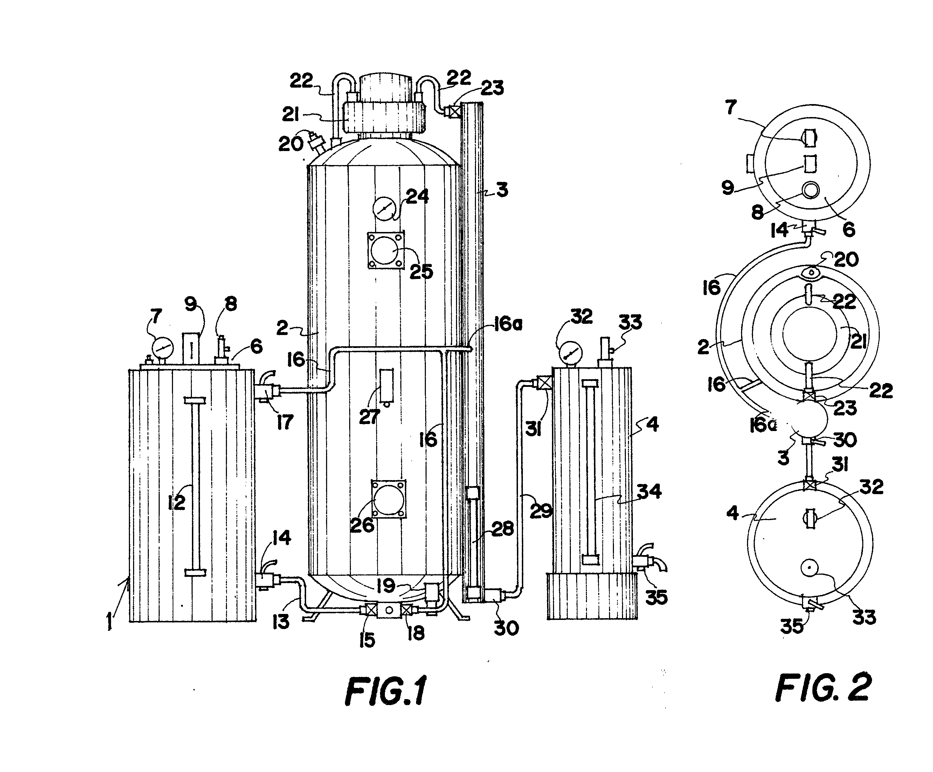

Referring to the drawings, particularly Figures 1 and 2, the system for producing alcohol consists of a sterilizer 1, a fermentation and distillation vat 2, a condenser 3 and an accumulator 4, with their connecting pipe lines.

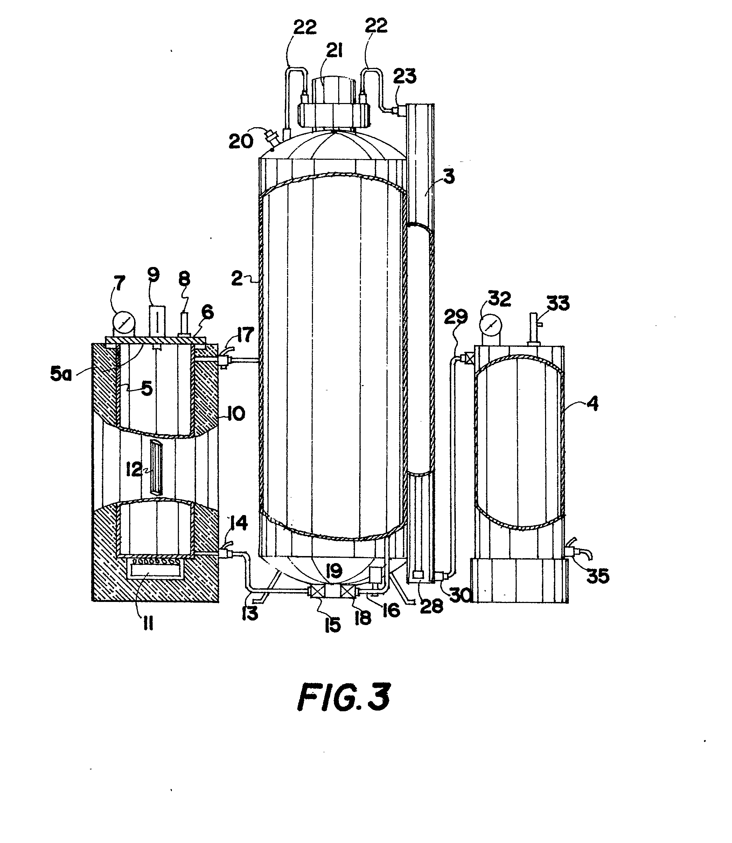

The sterilizer 1, as shown in Fig. 3, includes a cylindrical container 5 with loading part 5a at the top, a removable part oover 6 on which a pressure gauge 7, a relief valve 8 and a thermometer 9 are installed. The container 5 is enclosed in a cylindrical bousing 10 which is made of heat insulating material, such as asbestes or foam concrete, and beneath the bottom of the container 5 is a burner 11. The sterilizer 1 includes also a sight glass 12 whose inner space is understood to be in communication with the inner space of the container 5.

The container 5 of the sterilizer 1 and the fermentation and distillation vat 2, are in communication with each other through their bottom portions by means of the pipe line 13 which has a stop cock 14 and a check valve 15. The inner space of the upper portion of the container 5 is in communication with the inner space of the bottom portion of the vat 2 by means of the pipe line 16 which is likewise provided with a stop cocks 17 and 18 and a check valve 19. Connected to the pipe line 16 a pipe line 16a which leads to the inner space of the middle portion of the condenser 3.

The fermentation and distillation vat 2 is in the form of a cylindrical tank, and installed on its top are the relief valve 20 and a vacuum pump 21. The inner space of the upper portion of the vat 1 is in communication with the inner space of the upper end of the condenser 3, through the pipe line 22, the vacuum pump 21 and the check valve 23. Installed at the side of the vat 2 are the vacuum gauge 24, the sight glasses 25 and 26 and the thermometer 27.

The condenser 3 is in the form of a tube positioned vertically with its side welded to the side of the vat 2 and provided with a sight glass 28. Although it is not shown in the drawings, the outer surface of the conden-

0039518

ser 3 may be provided with cooling fins and its inner space may have steel wool to accelerate the condensation of alcohol. The condenser 3 has a pipe line 29 at its lower end which leads to the inner space of the upper portion of the accumulator 4. This pipe line is likewise provided with a stop cock 30 and a check valve 31.

Installed on the top of the accumulator 4 are the pressure gauge 32, the relief valve 33 and at its side is the sight glass 34 and installed at its lower side is the outlet cock 35.

The operation of the system starts at the sterilizer 1 where the raw materials are loaded to its container 5, through the loading port 5a after removal of the port cover 6. As soon as the container is sufficiently loaded with the raw materials, the port cover 6 is again placed on the top of the container 5 and the burner or heater 11 is put in operation. The thermometer 9 monitors the mash temperature to insure thoruugh sterilization. As the mash is heated, the air in the container 5 expands and when the pressure reaches pre-selected level, the relief valve 8 blows off to limit the pressure in the container 5 to pre-determined level, The temperature, and the content in the sterilizer 1 are respectively detected by the thermometer 9 and the sight glass 12, respectively.

When the mash loaded in the sterilizer is already properly sterilized, the mash is transferred from the sterilizer 1 to the fermentation and distillation vat 2 by starting the vacuum pump 21 so that is starts to reduce the pressure in the vat 2 while at the same time. increasing the pressure in the container 5 of the sterilizer 1 pushing the sterilizer mash thru the stop cock 14 and pipe 15 into the vat 2.

This operation is repeated until there is enough mash in the vat 2 to start fermentation. In the process of operation, if more mash is needed to replenish used up mash in the vat 2 the .ame can be added by the foregoing process provided the mash is cooled to the vicinity of the temperature in the mash at the vat 2 as indicated by the thermometer 27.

The fermentation agent is transferred to the vat 2 by loading as in the previous transfer operation of the sterilized mash, but in cold state. The agent is then mixed with the mash in the vat 2 by opening 19 and letting gas bubble to raise up and mix the mash in the vat 2. All the parts in the system can be inactive as fermentation taking place in the vat 2 builds up alcohol concentration in the mash therein, but as soon as alcohol approaches 10% concentration, the vacuum pump 21 starts operating. The vacuum pump 21 obviously reduces the pressure in the vat 2, thus accelerating the escape of alcohol from the mash in vapor form, and the vacuum pump 21 transfers said alcohol vapor into the condenser 3, thru the pipe line 22 and the check valve 23, and this raises the pressure in the condenser 3 allowing the alcohol vapor to condense therein and fall to the bottom.

Alcohol accumulates at the bottom of condenser 3 and transferred by pressure in the condenser 3 to the accumulator 4, through the valve 30, the pipe line 29 and the check valve 31, The accumulator is kept at a pre-determined pressure by the relief valve and monitored by the pressure gauge 32 as to operating pressure and by the sight glass 34 as to content.

Carbon dioxide which is the by-product of fermentation may be recovered at relief valve 33, which alcohol is drawn from the system through the outlet cock 35.

The vat 2 is monitored by the vacuum gauge 24 as to pressure and by the sight glasses 25 and 26 for the top and bottom leading limits, besides the thermometer 27 which monitors its operating temperature.

As the distillation progresses, the alcohol concentration at the top portion of the vat 2 is reduced while diffusion of the alcohol content of the mash at the bottom portion of the vat 2 is not fast enough to keep the alcohol concentration at the bottom portion of said vat. To prevent the concentration level of the alcohol in the mash to reach the 15% level which is fatal or destractive to the fermentation agent, gas under pressure at the condenser 3 is directed to the bottom of the vat 2, through the pipe lines 16a and 16, and allowed to bubble and mix the mash at periodic intervals as well as to absorb alcohol vapor for delivery to the top of the vat 2.

From the foregoing, the important advantage attained by the system is time, as the different steps previously compartmentalized in the conventional process steps can now be overlapped and made to take place simultaneously without interruption. This ability to combine process steps in one operation has reduced the container requirement as the fermentation and distillation take place in one vat only instead of two containers, and it totally eliminates the concentration step in the production of near anhydrous alcohol as the old system can only produce 65% alcohol.

Distillation under vacuum condition, one of the steps adopted in this process has many faceted advantages. One is that alcohol concentration in the mash can be controlled without killing the fermentation agent. Another is that the distillation under vacuum condition reduces mass temperature and thus inhibits the formation of acids which is detrimental to the end product, but one advantage that is noteworthy, is that it eliminates from the old process the need of another step for concentrating the product to arrive at a usable concentration as in this process, water is not distilled with the alcohol, as in the thermal distillation, and its product is already anhydrous or very much near it.

Having thus described in detail the embodiment of my invention it will be understood that the present em-

-8-

bodiment or structural arrangement is presented by way
of example and that changes and modifications may be
resorted to without departing from the spirit or scope
of the invention as outlined in the appended claims.

CLAIMS

1. A system for producing near absolute alcohol comprising a sterilizing means (1) in which raw materials are heated, said means having monitoring devices (7, 9, 12) to determine the pressure, temperature therein and the level of its content; a fermentation and distillation tank (2) having a vacuum pump (21) and monitoring devices (24 - 27) to determine the pressure and temperature therein and the level of its content, said tank being in communication with said sterilizing means (1) and adopted to receive from the latter the sterilized mash and store said mash while the same is undergoing simultaneous fermentation and distillation under vacuum condition to produce alcohol vapor within the upper space of said tank (2); a condenser (3) in communication with the upper and inner space of said tank (2) to transfer said alcohol vapor from said tank, upon operation of said pump (21) and convert said alcohol vapor into liquid alcohol; and a collecting means (4) in the form of a tank in communication with said condenser (3) and adopted to receive the liquid alcohol from said condenser (3) and to store the same, said collecting means (4) having a monitoring devices (32, 34) to determine the pressure and the level of the content therein, a relief valve (33) for discharging carbon dioxide and an outlet from which alcohol is drawn.

—

2. The system as claimed in claim 1, characterzied in that said sterilizing means (1) and said fermentation and distillation tank (2) are in communication with each other by means of a pair of pipe lines (13, 16), one (13) of which connects the lower part of said sterilizer (1) to the lower part of said tank (2), and the other (16) one connects the upper part of said sterilizing means (1) to the lower part of said tank (2).

3. The system as claimed in claims 1 and 2, characterized in that said fermentation and distillation tank (2) and said condenser (3) are in communication with each other by means of said vacuum pump (21) and a pipe line (22) with a check valve (23), and by means of a pipe line (16a) connected to the middle section of said condenser (3) and pipe line (16a) connected to said pipe line (16) leading to the lower portion of said tank (2).

4. A system as claimed in claim 1, characterized in that said condenser (3) being provided with a cooling fins at its outer surface and being provided further inside with steel wool to accelerate the condensation of alcohol.

0039518

5. A process for producing near absolute alcohol comprising heating the raw materials under controlled conditions to convert the same into sterilized mash; simultaneously fermenting and distilling said sterilized mash together with fermentation agent under partial vacuum condition to produce and accumulate alcohol vapor above the surface of the mash; through the mixing and absorbing action of carbon dioxide discharged at the bottom of the fermentation and distillation tank (2); condensing said alcohol vapor into liquid alcohol and collecting said alcohol after discharging the undesirable carbon dioxide content of the processed alcohol.

FIG.1

FIG.2

0039518

**FIG.3**

European Patent
Office

**EUROPEAN SEARCH REPORT**

0039518

Application number

EP 81 10 3420

## DOCUMENTS CONSIDERED TO BE RELEVANT

| Category | Citation of document with indication, where appropriate, of relevant passages | Relevant to claim |
|---|---|---|
| X | BIOTECHNOLOGY & BIOENGINEERING, vol. XIX, 1977, New York, US G.R. CYSEWSKI et al. "Rapid ethanol fermentations using vacuum and cell recycle", pages 1125-1143. <br><br> * Page 1125, lines 8,9; page 1127, lines 1-10; page 1128, figure 1 * <br><br> -- <br><br> US - A - 2 440 925 (B.C. BOECKELER) <br><br> * Claim 1; column 4, lines 5-23; figure 3 * <br><br> ---- | 1,5 <br><br><br><br><br><br><br><br><br><br> 1,5 |

**CLASSIFICATION OF THE APPLICATION (Int. Cl.³)**

C 12 P 7/06

**TECHNICAL FIELDS SEARCHED (Int. Cl.³)**

C 12 P 7/06

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 04-08-1981 | ALMOND |

EPO Form 1503.1  06.78